Europäisches Patentamt

European Patent Office

Office européen des brevets

(19)

(11) Veröffentlichungsnummer: **0 294 410**
**B1**

(12)

# EUROPÄISCHE PATENTSCHRIFT

(45) Veröffentlichungstag der Patentschrift: **14.11.90**

(21) Anmeldenummer: **87907962.2**

(22) Anmeldetag: **12.12.87**

(86) Internationale Anmeldenummer:
**PCT/EP87/00775**

(87) Internationale Veröffentlichungsnummer:
**WO 88/04663 30.06.88 Gazette 88/14**

(51) Int. Cl.⁵: **C 07 J 19/00, B 01 D 15/08**

(54) **VERFAHREN ZUR ANREICHERUNG UND/ODER ISOLIERUNG VON HERZGLYKOSIDEN UNTER VERWENDUNG UNPOLARER ADSORBERHARZE.**

(30) Priorität: **20.12.86 DE 3643760**

(43) Veröffentlichungstag der Anmeldung:
**14.12.88 Patentblatt 88/50**

(45) Bekanntmachung des Hinweises auf die
Patenterteilung:
**14.11.90 Patentblatt 90/46**

(84) Benannte Vertragsstaaten:
**BE CH DE FR GB IT LI**

(56) Entgegenhaltungen:
**FR-A-2 552 767**

**Chemical Abstracts,Band 101, Nr.12 ; 17.Sept.
1984 (Columbus, Ohio, US), siehe Seite 410,
Z'fassung 97633y .**

(73) Patentinhaber: **BOEHRINGER MANNHEIM
GMBH
Sandhofer Strasse 116
D-6800 Mannheim 31 (DE)**

(72) Erfinder: **ROSS, Carl, Heinz
Rathausstrasse 45
D-6806 Viernheim (DE)**
Erfinder: **MACHAT, Rudolf
Bassermannstrasse 65
D-6800 Mannheim 1 (DE)**
Erfinder: **HÄRING, Werner
Fichtenweg 3
D-6148 Heppenheim/Kirschhausen (DE)**
Erfinder: **LETTENBAUER, Gustav
Am Weingarten 1
D-6840 Lampertheim (DE)**

**Beschreibung**

Gegenstand der vorliegenden Erfindung sind Verfahren zur Aufarbeitung von Herzglykoside enthaltenden Mischungen, wie Rohextrakten, Produkten chemischer Abwandlungen und Kristallisationsstufen, sowie Mutterlaugen.

Unter Herzglykosiden werden Verbindungen der allgemeinen Formeln I, II oder III verstanden

$$R—O—(D—O—)_{1-3}-(\text{Glucoserest-O—})_{1-2}—H \qquad (I)$$

$$R—O—(D—O—)_1 \ oder_3—R' \qquad (II)$$

bzw.

$$R—O—(\text{Glucoserest-O—})_{1-2}—H \qquad (III)$$

worin jeweils

R ein Aglykon, R' Wasserstoff, eine Methyl- oder Acetylgruppe und D einen Desoxyzuckerrest bedeuten (vgl. Ullmann, 3. Aufl., Band 8, S. 222—231).

Zur Isolierung der Herzglykoside, wie Digoxin, Metildigoxin, Strophantinen, Lanatosiden aus Mischungen wie sie z.B. bei Extraktionen der Pflanzenteile, chemischen Abwandlungen und Kristallisationsoperationen anfallen, sind meist aufwendige Verfahren nötig, wie mehrfache Gegenstromverteilung, Umlösungen und Umfällungen. (Vergl. zum Beispiel: Ullmann, a.a.O.).

Diese Verfahren erfordern den Einsatz großer Mengen potentiell umweltgefährdender organischer Lösemittel wie Chloroform, Dichlormethan, Trichlorethylen. Die Handhabung dieser Lösemittel in großen Mengen führt zu einem großen technischen Aufwand zur Reinhaltung von Luft und Wasser. Die Verluste an wertvollen Glykosiden in nicht verwertbaren Mutterlaugen und Konzentraten sind hoch.

Hier will die Erfindung Abhilfe schaffen. Die Erfindung, wie sie in den Ansprüchen gekennzeichnet ist, löst die Aufgabe, Verfahren zur Anreicherung und Isolierung von Herzglykosiden aus Mischungen wie Rohextrakten, Produkten chemischer Abwandlungen und Kristallisationsstufen, sowie aus Mutterlaugen bereitzustellen, die weitestgehend ohne die nach dem Stand der Technik erforderlichen potentiell umweltgefährdenden Lösemittel auskommen und zudem die Verluste an wertvollen Glykosiden verringern und damit die Ausbeuten steigern.

Es wurde gefunden, daß die Anreicherung und Aufreinigung der Herzglykoside aus den oben näher bezeichneten Mischungen und Mutterlaugen durch Adsorption an unpolaren, großporigen Harzen aus wäßriger Lösung eventuell unter Zusatz von mit Wasser mischbaren Lösemitteln und anschließende Desorption mit Wasser-Lösemittelgemischen oder reinen Lösemitteln bewerkstelligt werden kann. Überraschend ist dabei insbesondere, daß dabei gleichzeitig eine Trennung der Glykosidgemische in Einzelverbindungen oder Gruppen durch Variation der Zusammensetzung der Desorptionslösung möglich ist.

Die Isolierung von wertvollen Stoffen, wie den antibiotisch wirksamen Cephalosporinen, aus stark verdünnten Lösungen mit Hilfe polymerer unpolarer Harze ist seit langem bekannt. Die Problematik der Anreicherung und Isolierung von Herzglykosiden aus komplexen Herzglykosid-Gemischen und insbesondere die Gefährdung durch die dazu erforderlichen Lösemittel ist noch länger bekannt. Die vorliegende Erfindung befriedigt damit ein lange bestehendes Bedürfnis.

Die zur erfindungsgemäßen Anreicherung und Isolierung erforderliche Adsorption und Desorption kann kontinuierlich oder diskontinuierlich (Batch-Verfahren) durchgeführt werden. Bevorzugt wird dieser Prozeß aber auf einer Säule unter leichtem Druck von 1 bis 10 bar bei Temperaturen von 20 bis 50°C, bevorzugt Raumtemperatur durchgeführt.

Dazu gibt man auf eine mit einem geeigneten Harz gefüllte Säule die wäßrige Lösung eines Rohextraktes oder verunreinigten Glykosidgemisches eventuell unter Zusatz von 1—80% niederen Alkoholen, vorzugsweise Methanol, Ethanol oder Isopropanol, niederen Ketonen, vorzugsweise Aceton, nachdem durch Behandlung des Roh-Extraktes mit einem unpolaren Lösemittel, vorzugsweise Hexan, Toluol, Isohexan oder Petrolether, die fettartigen Bestandteile entfernt worden sind.

Alle stark polaren Stoffe, wie Zucker, werden durch Spülen mit Wasser aus der beladenen Säule entfernt. Durch Zusatz von mit Wasser mischbaren Lösemitteln, vorzugsweise Alkoholen, niederen Ketonen oder cyclischen Ethern, durch Zugabe von Salzen und Einstellung eines bestimmten pH-Wertes wird die Elutionslösung so variiert, daß einzelne Herzglykoside oder Gruppen von Glykosiden aus dem Harz desorbiert werden. Alle Lösemittel können so gewählt werden, daß sie möglichst umweltverträglich sind. Die für die Vorreinigung erforderlichen großen Mengen chlorierter Kohlenwasserstoffe können dadurch umgangen werden.

Als Adsorbentien werden unpolare Adsorberharze mit großer Oberfläche und zwar großporige nichtionische Harze auf Basis vernetzter Styrolpolymere, Acrylesterpolymerisate und/oder Styrol-Divinylbenzol-Copolymerisate verwendet Harze, die chemisch modifiziert, insbesondere halogeniert oder physikalisch klassiert, wie gesiebt, in der Korngröße, Oberfläche vereinheitlicht, sein können. Die spezifische Oberfläche der verwendeten Harze beträgt vorzugsweise 100—1000 m²/g Harze.

Solche Harze sind z.B. unter den Bezeichnungen Amberlite XAD (Warenzeichen: Rohm and Haas Co.), Diaion HP (Warenzeichen: Mitsubishi Chemical Industries Limited) oder Sepabeads SP (Warenzeichen: Mitsubishi Chemical Industries Limited) jeweils in Serien mit verschiedenen Oberflächen- und damit Adsorptionseigenschaften erhältlich. Zum Beispiel:

|  | Chemische Struktur | Spezifische Oberfläche $m^2$/g Harz |
|---|---|---|
| Amberlite® XAD—2 | CAS-Reg.Nr.*) 9060—05—3 |  |
| Amberlite® XAD—4 | CAS-Reg.Nr.*) 37380—42—0 | 725 |
| Amberlite® XAD—7 | CAS-Reg.Nr.*) 37380—43—1 | 450 |
| Diaion® HP 10 |  | 501 |
| Diaion® HP 20 |  | 718 |
| Diaion® HP 30 |  | 570 |
| Diaion® HP 40 |  | 705 |
| Diaion® HP 50 |  | 590 |
| Sepabeads® SP 207 |  | 400 |

*) Chemical Abstracts Service Registernummer

Innerhalb dieser Serien wird noch nach Korngröße differenziert.

|  | Chemische Sturktur | Spezifische Oberfläche $m^2$/g Harz |
|---|---|---|
| Diaion® HP 20 SS | (wie Diaion® HP 20) |  |
| Diaion® HP 2MG |  | 550 |
| Diaion® HP 21 | (wie Diaion® HP 20) | 700 |
| und Diaion® HP 21 SS |  |  |

Die letztgenannten 4 Harze haben sich als besonders geeignet herausgestellt. Insbesondere aber Diaion® HP 20 SS. und HP 21 SS.

Die unterschiedlichen Adsorptionsfähigkeiten der verschiedenen Harztypen lassen sich zu besonders zweckmäßigen Trennverfahren kombinieren.

Weitere Pflanzeninhaltsstoffe, wie Chlorophyll, desorbiert man durch Aufgabe von organischen Lösemitteln, vorzugsweise Alkoholen, Ketonen oder Estern auf die Säule. Nach Einengen der so erhaltenen Lösungen werden reine Stoffe nach Umlösen und Umfällen eventuell unter Zusatz von Hilfsstoffen, vorzugsweise Aktivkohle, Aluminiumoxid oder Bleicherden (wie Floridin) erhalten. Alternativ zum energieaufwendigen Einengen kann die Isolierung aus wäßrigen Lösungen auch durch Aufziehen (Adsorption) auf ein polymeres Harz und Desorption mit einem organischen Lösemittel erfolgen.

Die Erfindung wird durch die folgenden Beispiele näher erläutert:

### Beispiel 1

Digoxin

Auf eine Säule, gefüllt mit 410 ml Diaion® HP 20 SS (Hersteller: Mitsubishi, Japan), werden 3,6 l wäßrig/methanolischer Rohextrakt von Digitalis lanata nach vorhergegangener Fermentation, Verseifung und Entfettung aufgezogen. Der Extrakt enthält 117 g Trockensubstanz mit einem Digoxingehalt von 1,95 g. Auf der Säule werden nach Spülen mit 4,9 l Wasser 12,2 g Trockensubstanz mit 1,95 g Digoxin festgehalten.

Nach Elution bei 3 bar mit wäßrigem Isopropanol erhält man Fraktionen die stark an Digoxin angereichert sind. 6,3 l 15% Isopropanol-Lössung werden zusammengeführt und bis auf 100 ml eingeengt. Die auskristallisierte Rohdigoxinfraktion (4,9 g), wird in 50 ml Methanol/Chloroform 1:1 heiß gelöst, mit Aktivkohle behandelt und über 7,5 g Aluminiumoxid filtriert. Man wäscht mit 80 ml Methanol/Chloroform 1:1, engt die gesammelten Filtrate auf 50 ml ein und fällt mit 65 ml Diisopropylether. Man isoliert 1,4 g Digoxin.

### Beispiel 2

Lanatosid C

20 g trockener Extrakt der genuinen Glykoside aus Digitalis lanata (Gehalt: Lanatosid C 12%, ca. 1% Dioxin, ca. 1,5% α-Acetyldigoxin) werden in 150 ml Wasser und 45 ml Methanol gelöst. Diese Lösung wird mit 1,4 l/h Fließgeschwindigkeit auf eine Säule mit 410 ml Diaion® HP 20 SS gebracht. Mit einem Druck von 4—5 bar wird anschließend mit Wasser, dem steigende Mengen Methanol zugesetzt werden, eluiert. Ab 50% Methanol-Anteil können die Glykoside in der Reihenfolge Lanatosid C, Digoxin, α-Acetyldigoxin, Lanatosid A im Eluat nachgewiesen werden. Die Fraktion von 6 l mit 55% Methanol wird vollständig eingeengt (2,53 g mit 60—70% Lanatosid C und 3% Digoxin) und aus Ethanol unter Zusatz von Aktivkohle umkristallisiert. Men erhält 1,0 g reines Lanatosid C. Der Mutterlaugenrückstand und Mischfraktionen werden erneut über die Säule gereinigt und getrennt.

### Beispiel 3

K-Strophantin

20 g Trockensubstanz des mit Hexan entfetteten Ethanolextraktes von Strophanthus kombé-Samen werden in 100 ml Wasser gelöst und auf eine Säule, gefüllt mit 410 ml Diaion® HP 20 SS gepumpt.

Mit 4,2 l Wasser werden bei 4—5 bar und einer Fließgeschwindigkeit von 1,4 l/h alle Nichtglykoside ausgewaschen (8,3 Trockensubstanz).

Nach Zusatz von 45% Methanol zur Elutionsflüssigkeit erhält man 7,8 l Flüssigkeit mit den Glykosiden. Es wird eingeengt (6,9 g), in Ethanol gelöst, mit Aktivkohle behandelt und mit Diisopropylether gefällt. Es werden 6,06 g reines Glykosid-Gemisch (75% γ-K-Strophantin, 12,5% β$_1$-K-Strophantin, 9,5% β$_2$-K-Strophantin) erhalten. Das Gemisch entspricht ohne größere Weiterbearbeitung bereits den Anforderungen der Pharmacopoeen.

### Beispielen 4

γ-K-Strophantin und β$_2$-K-Strophantin

20 g Trockensubstanz wie aus Beispiel 3 werden auf 410 ml Diaion® HP 20 SS aufgebracht. Man eluiert die Säule mit Wasser mit einem bis auf 30% steigenden Methanolgehalt. Die so erhaltenen Fraktionen (Trockensubstanz: 10,1 g) enthalten keine Glykoside des K-Strophantins. Die Fraktion (2,8 l) mit 40% Methanol ergibt nach Einengen 5,3 g γ-K-Strophantin. Nach Erhöhen des Methanolanteiles auf 45% wird eine Mischfraktion (7,5 l) (3,0 g nach Einengen) von γ-, β$_1$- und β$_2$-K-Strophantin erhalten, 2,1 l Eluat mit 50 prozentigem Methanol enthalten 0,61 g (Trockensubstanz) β$_2$--K-Strophantin.

### Beispiel 5

g-Strophantin

Mutterlauge von Ethanol-Extrakten von Strophantus gratus-Samen die nicht mehr zur Kristallisation zu bringen ist, aber noch Glykosid enthält, wird zur Trockene gebracht.

20 g Trockensubstanz werden in 100 ml 10prozentigem Methanol gelöst und mit 0,9 l/h Fließgeschwindigkeit auf eine Säule mit 410 ml Diaion® HP 20 SS gepumpt. Man eluiert mit 4 l 10prozentigem Methanol alle Zuckerbestandteile und polare Nichtherzglykoside.

Nach Erhöhen des Methanolgehaltes auf 20% spült man mit 3 l bei 3—4 bar und einer Flußrate von 1,4 l/h g-Strophantin (3,69 g) aus der Säule. Nach Einengen wird aus Wasser unter Zusatz von Aktivkohle umkristallisiert. Man erhält 2,12 g reines g-Strophantin.

### Beispiel 6

Digoxin (vgl. Zeichnung)

60 g Trockenextrakt (enthält ca. 1,0 g Digoxin) — hergestellt durch wäßrig/methanolische Extraktion von Digitalis lanata, Fermentation, Verseifung, Entfettung und Trocknen — werden in 120 ml Methanol und

4

EP 0 294 410 B1

480 ml Wasser gelöst, mit konzentrierter Ammoniaklösung auf pH 7.5 eingestellt und mittels der Pumpe (3) auf die Säule (1), gefüllt mit 410 ml Diaion® HP 2MG, gepumpt. Ventil (6) ist auf Ablauf (8) gestellt. Nach Spülen mit 2,2 l Wasser und 3,8 l Methanol/Wasser (v./v. 25:75) werden im Ablauf nach Einengen 54.8 g Trockensubstanz erhalten, die keine Herzglykoside enthalten. Die Ventile (6) und (5) werden nun so gestellt, daß der Ablauf von Säule (1) auf Säule (2), gefüllt mit 50 ml Diaion® HP 20 SS, führt. 5 l Elutionslösung Methanol/Wasser (v./v. 45:55) enthalten im Ablauf (7) kein Digoxin. Fraktionen mit Nebenglykosiden können getrennt aufgearbeitet werden. Ventile (4) und (5) werden nun so gestellt, daß die Elutionslösung direkt auf Säule (2) gegeben wird. Nach 1 l eines Methanol/Wasser-Gemisches (v./v. 50:50), die 0,9 gTrockensubstanz mit 27% Digoxin enthalten und in den Trennprozeß zurückgeführt werden, wird mit 1,5 l eines Methanol/Wasser-Gemischs (v./v. 55:45) eluiert. Diese Fraktion wird eingeengt, aus Methanol/ Wasser umgelöst, in Methanol/Chloroform gelöst, mit Aktivkohle und Aluminiumoxid behandelt und mit Diisopropylether gefällt. Man isoliert 684 mg Dioxin hoher Reinheit.

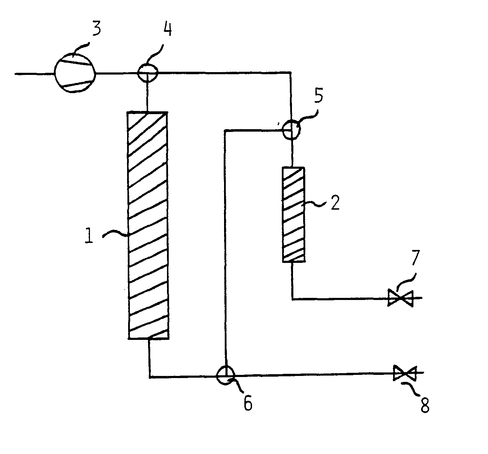

Das Verfahren des Beispiels 6 wird anhand der Zeichnung weiter erläutert. Sie zeigt lediglich eine mögliche Ausgestaltung des Flußdiagrammes.

Darin bedeuten die Bezugszeichen
(1) Säule gefüllt mit 410 ml Diaion® HP 2MG
(2) Säule gefüllt mit 50 ml Diaion® HP 20 SS
(3) Pumpe
(4) 3-Wege-Ventil
(5) 3-Wege-Ventil
(6) 3-Wege-Ventil
(7) Ablauf-Ventil
(8) Ablauf-Ventil

Beispiel 7

Metildigoxin

10 g des aus der Methylierung von Digoxin mit Dimethylsulfat erhaltenen Gemisches aus hauptsächlich β-Metildigoxin (60—65%) α-Metildigoxin (ca. 7,5%), Digoxin (15—20%) und Dimethylethern des Digoxins (ca. 8%) werden in 85 ml Ethanol abs. und 12 ml Wasser warm gelöst und klar filtriert. Diese Lösung wird auf eine Säule mit 410 ml HP 20 SS, die zuvor mit Ethanol 30% gespült wurde, gepumpt. Anschließend wird mit Wasser mit steigendem Ethanolgehalt unter einem Druck von 4 bar und einem Durchfluß von ca. 1 l/Stunde gespült.

4,75 l Eluat mit 40% Ethanol enthalten 3,91 g Feststoff. Das Gemisch enthält alles nicht umgesetzte Digoxin α-Metildigoxin und β-Metildigoxin und kann erneut auf der Säule getrennt werden. Die folgende Fraktion von 5 l enthält 4,73 g schwach verunreinigtes β-Metildigoxin. Wird der Ethanolgehalt erhöht, werden Diether und höhere Ether des Digoxins eluiert. Die Hauptfraktion mit Metildigoxin wird aus Methanol/Wasser umgefällt, mit Chloroform aufgekocht und mit Aceton ausgerührt.

Man erhält 3,65 g β-Metildigoxin von Standardqualität.

Beispiel 8

Digoxin

120 g Trockenextrakt mit einem Digoxin-Gehalt von ca. 1,9 g (vgl. Beispiel 6) werden in 960 ml Wasser, 240 ml Ethanol und 5 ml konzentrierter Ammoniaklösung bei Raumtemperatur gelöst und klar filtriert. Die Lösung wird auf eine Säule mit 410 ml Diaion® HP 20 gepumpt.
(Druck 2 bar, Flußrate 1—1,2 l/Stunde).
Säulen ablauf und 1,8 l Spülwasser enthalten 87,2 g Trockensubstanz frei von Herzglykosiden.
Es folgen Elutionen mit 4,4 l Ethanol 20% und 2,4 l Ethanol 25,5%. Letztere enthält geringe Ansteile an Digoxin (<2,5%) und wird erneut eingesetzt.

5,0 l Eluat Ethanol 40% enthalten die Hauptmenge an Digoxin (8,3 Trockensubstanz, ca. 20% Digoxin). Nach einer Zwischenfraktion (1,2 l) Ethanol 43%, die zurückgeführt wird, wird mit reinem Ethanol die Säule für den nächsten Zyklus gespült. In den ersten 2 l des Spülethanols findet man die Hauptmenge des Digitoxins (Trockenrückstnad 3,5 g, 10% Digitoxin), das daraus gewonnen werden kann. Die Digoxin-Fraktion wird durch Destillation auf 1,42 l konzentriert und auf eine Säule mit 410 ml HP 20 SS gepumpt (Druck 4—10 bar, Fließgeschwindigkeit 1,2 l/Stunde). Der Säulenablauf von 1,5 l und die Fraktionen mit Ethanol 20% (1,5 l), Ethanol 24,5% (4,6 l) und Ethanol 27% (3,75 l) enthalten kein Digoxin. Nach einer Spülung mit Ethanol 33% (0,5 l) zur Abtrennung von Digoxigenin-bis-digitoxosid wird der Ethanolgehalt auf 40% gesteigert.

Man sammelt 6 Fraktionen zu 0,39 l. Die Fraktionen 1,5 und 6 werden zur Elution der HP 20 Säule eingesetzt. In 3,72 g Trockensubstanz der Eluate 2,3 und 4 sind 1,86 g Digoxin enthalten. Zur Regenerierung wird die Säule mit reinem Ethanol gespült. Das rohe Digoxin wird in 50 ml Ethanol/Wasser 1:1 warm gelöst, auf 25 ml konzentriert, gekühlt und abgesaugt. Man erhält 1,85 g, die in Chloroform/Methanol 9:1 gelöst, mit Kieselgel und Aktivkohle behandelt und konzentriert werden. Das Kristallisat wird in Ethanol unter Zusatz von Ammoniak aufgekocht, heiß abgesaugt und getrocknet. Es werden 1,1 g Reindigoxin erhalten.

### Beispiel 9

Digoxin, Gitoxin, Digitoxin

Man löst 50 g entfetteten Trockenextrakt von Digitalis lanata (wie in Beispiel 6) in 415 ml Wasser, 154 ml Methanol und 2 ml konzentrierte Ammoniaklösung. Die klar filtrierte Lösung wird auf eine Säule mit 470 ml Diaion® HP 21 SS gepumpt. Mit einem Druck von 4 bar und einer Flußrate von 1,0 l/Stunde wird anschließend fraktioniert eluiert.

Säulenabläufe bis 55% Methanol (1,59 l) enthalten in 43,4 g Trockensubstanz nicht identifizierte Zucker und Glykoside.

Die letzten Fraktionen enthalten Digoxingenin-bis-digitoxosid stark angereichert und können auf dieses Glykosid aufgearbeitet werden.

2,5 l Eluat mit 65% Methanol-Anteil enthalten 1,12 g Trockensubstanz mit über 60% Digoxin, die nach üblichen Reinigungsschritten 0,52 g reines Digoxin ergibt. In den Fraktionen mit 70% Methanol (2,5 l) sind geringe Mengen Digoxin. Diese werden erneut auf die Säule gegeben.

1,5 l Fraktion (80% Methanol) enthalten stark angereichert das Glykosid Gitoxin und können auf dieses aufgearbeitet werden. 0,81 g der Fraktion mit reinem Methanol (0,9 l) enthalten 80 mg Digitoxin, das aus dieser Fraktion isoliert werden kann.

### Patentansprüche

1. Verfahren zur Anreicherung und/oder Isolierung von Herzglykosiden durch Flüssigkeitsverteilungschromatographie dadurch gekennzeichnet, daß Adsorption und Desorption an unpolaren, nichtionischen großporigen Harzen auf Basis von vernetzten Styrolpolymeren, Acrylesterpolymerisaten und/oder Styrol-Divinylbenzol-Copolymerisaten erfolgen.

2. Verfahren nach Anspruch 1 dadurch gekennzeichnet, daß die Harze eine spezifische Oberfläche von 100—1000 m²/g Harz haben.

3. Verfahren nach Anspruch 1 und 2 d.g., daß als Elutionsmittel Mischungen aus Wasser und niederen Alkoholen oder Ketonen im Verhältnis 99:1 bis 20:80 eingesetzt werden.

4. Verwendung von unpolaren, nichtionischen, großporigen Harzen auf Basis von vernetzten Styrolpolymeren Acrylesterpolymerisaten und/oder Styrol-Divinylbenzol-Copolymerisaten zur Anreicherung und/oder Isolierung von Herzglykosiden.

### Revendications

1. Procédé pour enrichir et/ou isoler des glycosides cardiotoniques par chromatographie de partage en phase liquide, caractérisé en ce que l'adsorption et la désorption sont réalisées sur des résines non polaires, non ioniques, à pores larges, à base de polymères styréniques réticules, de polymères d'esters acryliques et/ou de copolymères styrènedivinylbenzène.

2. Procédé selon la revendication 1, caractérisé en ce que les résines présentent une surface spécifique de 100—1000 m²/g de résine.

3. Procédé selon la revendication 1 ou 2, caractérisé en ce que comme éluant, on utilise des mélanges d'eau et d'alcools ou de cétones inférieurs, dans un rapport de 99:1 à 20:80.

4. Utilisation de résines non polaires, non ioniques, à pores larges, à base de polyères styréniques réticulés, de polymères d'esters acryliques et/ou de copolymères styrène-divinylbenzène, pour enrichir et/ou isoler des glycosides cardiotoniques.

### Claims

1. Process for the enrichment and/or isolation of heart glycosides by liquid partition chromatography, characterised in that adsorption and desorption take place on non-polar, non-ionic, large-pored resins based on cross-linked styrene polymers, acrylic ester polymers and/or styrene-divinylbenzene co-polymers.

2. Process according to claim 1, characterised in that the resins have a specific surface of 100—1000 m²/g.

3. Process according to claims 1 and 2, characterised in that, as elution agents, there are used mixtures of water and lower alcohols or ketones in the ratio of 99:1 to 20:80.

4. Use of non-polar, nono-ionic, large-pored resins based on cross-linked styrene polymers, acrylic ester polymers and/or styrene-divinylbenzene co-polymers for the enrichment and/or isolation of heart glycosides.